# EUROPÄISCHE PATENTSCHRIFT

(11) **EP 2 986 952 B1**
(45) Veröffentlichungstag und Bekanntmachung des Hinweises auf die Patenterteilung: **04.11.2020**
(21) Anmeldenummer: 14709530.1
(22) Anmeldetag: 20.01.2014
(51) Int. Cl.: G01F 11/28

(54) **VORRICHTUNG ZUM EINBRINGEN EINER DEFINIERTEN MENGE EINES ZWEITEN PULVERS IN EINEN PROZESSBEHÄLTER**
DEVICE FOR INTRODUCING A DEFINED QUANTITY OF A SECOND POWDER INTO A PROCESS VESSEL
DISPOSITIF D'INTRODUCTION D'UNE QUANTITÉ PRÉCISE D'UNE DEUXIÈME POUDRE DANS UN CONTENEUR

(30) Priorität: 19.04.2013 DE 102013104003
(43) Veröffentlichungstag der Anmeldung: 24.02.2016
(73) Patentinhaber: Glatt Systemtechnik GmbH, 01277 Dresden (DE); Harro Höfliger Verpackungsmaschinen GmbH, 71573 Allmersbach im Tal (DE)
(72) Erfinder: PRITZKE, Heinz, 01737 Kesselsdorf (DE); KNORR, Wolfgang, 01257 Dresden (DE); WURST, Reiner, 71549 Auenwald (DE)
(74) Vertreter: Hofmann, Klaus
(86) Internationale Anmeldenummer: PCT/DE2014/100012
(87) Internationale Veröffentlichungsnummer: WO 2014/169897

(56) Entgegenhaltungen:
- EP-A1- 2 184 590
- EP-A1- 2 508 219
- DE-B3-102006 061 992
- US-A1- 2011 212 519

## Beschreibung

Die Erfindung betrifft eine Vorrichtung zum Einbringen einer definierten Menge eines zweiten Pulvers in einen Prozessbehälter, in dem sich ein erstes Pulver oder ein Pulvergemisch befindet, wobei die Vorrichtung den Prozessbehälter (1), eine rohrförmige Kartusche (4) und eine Transporteinheit umfasst, wobei das zweite Pulver in die rohrförmige Kartusche (4) einbringbar ist, und wobei die Kartusche (4) verschiebbar in der Transporteinheit lagerbar ist. Die Vorrichtung ist insbesondere zum Eintrag von kleinen und kleinsten Mengen an pulverförmigen Stoffen in größere Prozessbehälter vorgesehen, insbesondere der Zumischung von geringen Mengen an pulverförmigen Arzneimittel-Aktivstoffen zu in einem Prozessbehälter bereits befindlichen Hauptkomponenten, wie beispielsweise Lactose oder Maisstärke. Die Erfindung ist besonders für Containmentanwendungen von Aktivstoffen und/oder Sterilanwendungen geeignet.

Nach dem Stand der Technik erfolgt die Zugabe von bestimmten Komponenten in Mischbehälter bekannterweise durch einfaches Einfüllen über eine Deckelöffnung. Dazu kann ein in der DE-OS 21 43 403 beschriebenes Taschendosiergerät für rieselfähiges Gut verwendet werden. Das Handdosiergerät wird wegen seiner hohen Toleranzen lediglich zur dosierten Abgabe von künstlichem Süßstoff verwendet, obwohl es für pulverförmige Arzneimittel vorgesehen war.

Eine weitere Vorrichtung zum Mischen eines Gemenges von pulverisierten Bestandteilen wird beispielsweise in DE 3 104 062 A1 beschrieben. In das Wirbelbett eines ersten Pulvers wird ein zweites Pulver eingeleitet. Dies erfolgt unter Druck durch Aufprall des zweiten pulverisierten Bestandteils auf eine innerhalb des Wirbelbettes zur gleichmäßigen Verteilung des zweiten Bestandteiles darin angeordnete Ablenkplatte.

Eine nach dem Schwerkraftprinzip wirkende Dosiervorrichtung ist beispielsweise in DE 2 917 189 B2 beschrieben. Zum schubweisen volumetrischen Dosieren von fließfähigen Pulvern mit einem Vorratsbehälter und einem aus einem Kolbenschieber mit einem Ober- und einem Unterkolben bestehenden Doppelkolbendosierer, dessen Unterkolben sich vom Oberkolben entfernen kann, ist dadurch gekennzeichnet, dass sich bei der Aufwärtsbewegung des Kolbenschiebers der Unterkolben vom Oberkolben entfernt und bei der Abwärtsbewegung des Kolbenschiebers dem Oberkolben nähert, wodurch sich das Volumen der zwischen dem Ober- und dem Unterkolben befindlichen Einschnürung auf ein vorgegebenes Maß bei der Aufwärtsbewegung vergrößert und bei der Abwärtsbewegung vor dem Passieren der Dosierkammer auf das vorgegebene Dosiervolumen verringert.

Eine weitere Möglichkeit der Zugabe besteht darin, dass der Eintrag über eine Deckelöffnung mittel langen Mischlanzen erfolgt, die bis in das Mischbett reichen.

In der DE 20 62 513 B2 ist eine Vorrichtung zum schubweisen volumetrischen Dosieren fließfähiger Pulver beschrieben, die einen vertikal angeordneten und mit einer Einschnürung versehenen Kolbenschieber als Zuteiler aufweist. Dosiervorrichtungen dieser Art haben den Nachteil hoher Toleranzen der dosierten Pulvermengen.

Zur Erfüllung einer allgemeinen Anforderung an Rohrkupplungen, dass die jeweiligen Hälften der Kupplung jeweils ein Ventil enthalten, das geöffnet werden kann, wenn die Kupplungshälften ordnungsgemäß miteinander verbunden sind und das zu einem vollständig geschlossenen Zustand bewegt werden muss, bevor die jeweiligen Hälften der Kupplung voneinander getrennt werden können, wird in EP 0 447 023 B1 eine Trockentrennkupplung für Leitungen mit 2 getrennt voneinander betätigbaren Ventilklappen beschrieben.

In DE 20 2009 018 738 U1 wird ein leicht lösbares Kupplungssystem zur axialen Kupplung von zwei parallelen Flanschen offenbart, bei dem die Stirnflächen der beiden Flansche zum Kuppeln axial aneinander gepresst werden und bei dem zwei Kupplungshälften drehbar gelagerte Schließklappen aufweisen, welche in der gekuppelten Position flächig aneinander liegen und gemeinsam geöffnet werden können.

In DE 4 342 962 C1 wird eine Vorrichtung zum Kuppeln von zwei jeweils einen Anschluss-Rohrstutzen aufweisenden Behältnissen beschrieben, bei der jeder der Rohrstutzen nahe seinem dem ihn tragenden Behältnis abgewandten Ende eine um einen Durchmesser des Rohrstutzens um im Wesentlichen 90° schwenkbare, mit ihrem Außendurchmesser im Wesentlichen dem Innendurchmesser des Rohrstutzens entsprechende Schließklappe aufweist.

Aus der DE 20 2005 015 569 U1 ist eine Zuschlagsstoff-Dosiervorrichtung für eine Polyurethan-Anlage zum Einbringen einer definierten Menge eines zweiten Stoffes in einen Prozessbehälter, in dem sich ein erster Stoff oder ein Stoffgemisch befindet bekannt, wobei der Stoff in eine in eine Aufnahme eingesetzte, mit einer Schnellkupplung ausgestatteten Kartusche 10 eingebracht und mittels eines Förderkolbens 26 entleert werden kann. Bei der Herstellung von Arzneimitteln besitzen die zu mischenden Komponenten sehr große Mengenunterschiede. Zu den Hauptkomponenten müssen geringste Mengen (0,01 bis 1%) an pulverförmigen Arzneimittel-Aktivstoffen in einer exakt definierten Dosierung zugemischt werden.

Sollen derart geringe Mengen zugemischt werden, besteht die Gefahr, dass bei der Zugabe entstehende Verluste bereits zu einer prozentual sehr hohen Abweichung des Mischungsverhältnisses führen. Diese Verluste können im Prozessbehälter selbst oder an den Dosiervorrichtungen entstehen. Bei einer Zugabe mittels einer offenen Dosiervorrichtung von oben in den Prozessbehälter können sich pulverförmige Stoffe an den Seitenwänden und anderen im Prozessbehälter befindlichen Flächen niederschlagen. An den Dosiervorrichtungen selbst können Verluste durch große Flächen oder lange Hubwege, wie beispielsweise bei den oben beschriebenen Dosierlanzen, entstehen. Sollen beispielsweise 20g an Aktivstoffen in einem 60I fassenden Prozessbehälter mit ca. 20kg enthaltenen Hauptkomponenten zugemischt werden, würde ein Verlust von 2g bereits zu unzulässigen Mindereinträgen von 10% an Aktivstoffen führen. Deshalb ist es vorteilhaft, wenn der Eintrag auf kürzestem Weg möglichst direkt in das Mischbett, also unter dem Füllstand im Prozessbehälter erfolgt.

EP 2 184 590 A1 beschreibt eine Dosiervorrichtung mit einer Wechselvorrichtung für Dosiereinheiten oder Funktionseinheiten. Eine Dosiervorrichtung weist einen Grundrahmen, mindestens eine Aufnahmevorrichtung zur Aufnahme einer beliebigen, in die Aufnahmevorrichtung einsetzbaren Dosiereinheit oder Funktionseinheit, eine Halterung und mindestens eine Antriebsvorrichtung auf. Die Halterung weist mindestens einen Halteplatz für eine Dosiereinheit oder Funktionseinheit auf. In einer unteren horizontalen Ebene des Grundrahmens ist ferner eine Wägezelle angeordnet, welche einen Lastaufnehmer zur Aufnahme eines Zielgefässes aufweist.

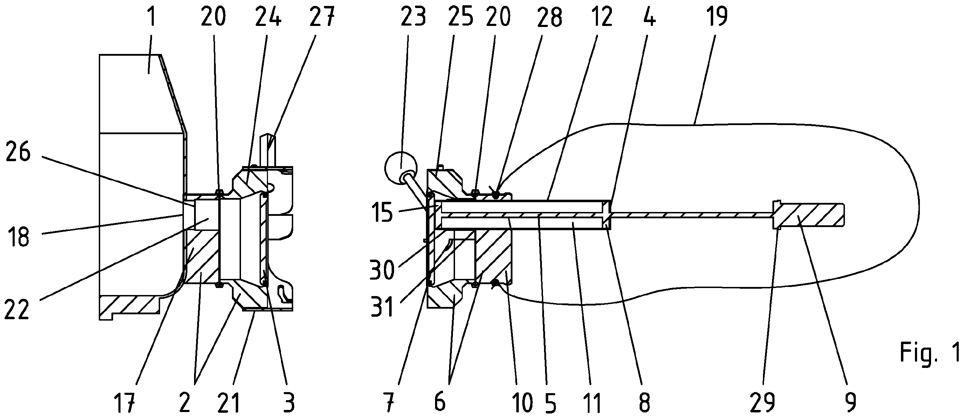

In Figur 1 wird eine Vorrichtung zum Einbringen einer definierten Menge eines zweiten Pulvers (Absatz 30) in einen Prozessbehälter (132) offenbart, in dem sich ein erstes Pulver oder Pulvergemisch befindet, wobei das zweite Pulver in eine rohrförmige Kartusche (140) eingebracht werden kann, und die Kartusche (140) verschiebbar in einer Transporteinheit (101, 104) gelagert werden kann.

Der Erfindung liegt als Aufgabe zugrunde, eine Vorrichtung anzugeben, mit der ein verlust- und kontaminationsfreier Eintrag von sehr kleinen Mengen an pulverförmigen Stoffen in größere Prozessbehälter ermöglicht wird, wobei Umgebungseinflüsse zu vermeiden sind. Der Eintrag soll mit einfachen Mitteln und einer hohen Dosiergenauigkeit erfolgen. Die Erfindung soll besonders für Containmentanwendungen von Aktivstoffen und/oder Sterilanwendungen geeignet sein.

Die Erfindung löst die Aufgabe durch die im Anspruch 1 angegebenen Merkmale. Vorteilhafte Weiterbildungen der Erfindung sind in den Unteransprüchen angegeben und werden nachstehend zusammen mit den beschriebenen Ausführungen der Erfindung, einschließlich der Zeichnungen, näher erläutert.

Das Einbringen einer definierten Menge eines zweiten Pulvers in einen Prozessbehälter, in dem sich bereits ein erstes Pulver oder ein Pulvergemisch befindet, wobei die Vorrichtung den Prozessbehälter, eine rohrförmige Kartusche und eine Transporteinheit umfasst wobei das zweite Pulver in die rohrförmige Kartusche einbringbar ist, und wobei die Kartusche verschiebbar in der Transporteinheit lagerbar ist, erfolgt durch einen am Prozessbehälter vorhandenen Kupplungsflansch, der eine Verschlussklappe aufweist.

Die Transporteinheit weist einen Verbindungsflansch mit einer Verschlussklappe auf, wobei der Verbindungsflansch mit dem Kupplungsflansch derart verbunden werden kann, dass die Verschlussklappe des Prozessbehälters und die Verschlussklappe der Transporteinheit geöffnet werden können. Die Kartusche kann innerhalb der Transporteinheit durch die geöffneten Verschlussklappen hindurch verschoben werden. Die vordere Entleerungsöffnung der Kartusche oder das Ende eines enthaltenen Förderkolbens sollte vorzugsweise bis in die Ebene der inneren Wandung des Prozessbehälters reichen. Das zu dosierende zweite Pulver kann dann mittels des Förderkolbens der Kartusche aus der Kartusche in den Prozessbehälter entleert werden.

In einer vorteilhaften Ausführung kann der Kupplungsflansch aus einem Anschlussstück und einem Klappenventil bestehen. Beide Teile können fest oder lösbar miteinander verbunden sein.

In einer weiteren vorteilhaften Ausführung kann der Verbindungsflansch aus einer Kartuschenaufnahme und einem Klappenventil bestehen. Beide Teile können ebenfalls fest oder lösbar miteinander verbunden sein.

In einer Ausführung der Erfindung besteht die Kartusche aus einer Kartuschenhülse, in deren inneren Pulverkammer der Förderkolben förderseitig mittels einer betätigbaren Kolbenstange verschiebbar angeordnet ist. Die Kartusche ist behälterseitig durch einen auf der gleichen Kolbenstange verschiebbar angeordneten Verschlusskolben gemeinsam verschließbar.

Bei einer weiteren Ausführung der Erfindung besteht die Kartusche aus einer Kartuschenhülse, in deren inneren Pulverkammer ein Doppelkolben verschiebbar angeordnet ist. Der Förderkolben kann dabei mittels einer Kolbenstange 1 verschoben werden, wogegen die Kartusche behälterseitig durch ein mittels einer zweiten Kolbenstange 2 verschiebbar angeordneten Verschlusskolben verschließbar ist. Beide Kolben können getrennt voneinander betätigt werden.

Eine besondere Ausführung der Erfindung betrifft eine Kartusche, die aus einer Kartuschenhülse besteht, in der ein um mindestens 100° axial verdrehbarer Kartuschenkern angeordnet ist. Über die gesamte Länge des Kartuschenkernes erstreckt sich eine Pulverkammer, in die ein Förderkolben mittels einer Kolbenstange verschiebbar angeordnet ist. Die Kartuschenhülse weist behälterseitig eine Entleerungsöffnung auf. Zum Entleeren der Kartusche ist die Pulverkammer über die Entleerungsöffnung so weit drehbar, dass die beiden Öffnungen so weit korrespondieren, bis das Pulver in den Prozessbehälter entleert werden kann.

Eine bevorzugte Ausführung wird in einer Vorrichtung realisiert, bei der die Kartusche aus einer Kartuschenhülse besteht, in die seitlich ein Drehverschluss integrierbar ist, mit dem eine Entleerungsöffnung der Kartusche mittels eines Betätigungshebels verschließbar ist. Die Kartuschenhülse ist dabei vorzugsweise oval ausgebildet. Diese Ausführung hat den Vorteil, dass kein gesonderter Verschlusskolben erforderlich ist.

Bei allen Ausführungen wird die Produktmenge, die aus der Kartusche dosiert oder entleert werden kann, über den Hub und den Durchmesser der Pulverkammer bestimmt. Für eine sichere Befüllung der Kartusche mit einer definierten Menge an Pulvermaterial kann die gesamte Transporteinheit entnommen und an einem gesonderten Ort transportiert werden. Am Verbindungsflansch sollte deshalb eine flexible Schutzhülle so befestigt sein, dass die Schutzhülle die eingeschobene Kartusche vollständig umschließt. Zweck ist, dass das System auch dann geschlossen bleibt, wenn die Kartusche vor Befüllung aus der Transporteinheit entnommen wird und dass keine besondere Abdichtung zwischen der Kartusche und der Kartuschenaufnahme erforderlich ist.

Zum Einbringen des zu dosierenden Pulvers werden der Verbindungsflansch der Transporteinheit und der Kupplungsflansch miteinander verbunden, wobei deren jeweiligen Verschlussklappen zueinander dicht abschließen und gemeinsam schwenkbar sein sollten. Damit bleiben die Außenseiten der Verschlussklappen pulverfrei. Dadurch wird die Operatorsicherheit im Sinne von Containment gesichert.

Statt eines Verschlusskolbens kann die Kartusche an ihrer Entleerungsöffnung mit einer durchstoßbaren Membran verschlossen sein. Vorzugsweise besteht die Membran aus einem Material, welches eine der Komponenten der im Prozessbehälter zu mischenden Pulver ist. Wäre die Membran aus anderen Stoffen, wie Papier oder Kunststoffen, muss gesichert werden, dass keine Fremdstoffe in den Prozessbehälter eintragbar sind, die zu ungewünschten Zusätzen oder Verschmutzungen führen könnten.

Insbesondere für Containmentanwendungen von Aktivstoffen oder Sterilanwendungen ist eine automatisierte Verfahrensführung vorteilhaft. Dazu kann die Vorrichtung so ausgebildet werden, dass der Förderkolben über eine lösbare Verbindung mit einem Antrieb gekoppelt ist, der eine automatische Dosierung oder Entleerung bewirkt. Der Antrieb kann ein elektrischer oder servopneumatischer Antrieb sein.

Durch die Verwendung von kleinen Kartuschen mit einer genau definierten Menge an Inhaltsstoffen wird eine präzise Mikrodosierung ermöglichst. Der mögliche Eintrag aus der Kartusche direkt unter den Füllstand des Mischbettes gewährleistet eine verlustfreie Zugabe, ohne dass sich Pulvermaterial an den Wänden absetzen kann. Durch die konstruktive Ausbildung der Zugabevorrichtung wird gewährleistet, dass auch innerhalb der Vorrichtung keine Ablagerungen oder Anhaftungen entstehen können. Die Vorrichtung ist prädestiniert für Containmentanwendungen von Aktivstoffen und/oder Sterilanwendungen.

Die Erfindung wird nachstehend an mehreren Ausführungsbeispielen näher erläutert. Zugehörig zeigen:
- Fig. 1: Schnittdarstellung einer erfindungsgemäßen Vorrichtung mit einer Anschlusseinheit und einer abgekuppelten Transporteinheit, in die eine Kartusche bis zu der noch geschlossenen Verschlussklappe geschoben ist,
- Fig. 2: Vorrichtung mit einer Doppelkolbenlösung,
- Fig. 3a - 3d: Vorrichtung mit einer Doppelkolbenlösung in 4 verschiedenen Positionen,
- Fig. 4: Kartusche mit einem verdrehbaren Kartuschenkern,
- Fig. 5: Kartusche mit einem Drehverschluss
- Fig. 6a, 6b: Perspektivdarstellung einer Vorrichtung mit einem Drehverschluss
- Fig. 7: Darstellung der Kartuschenbefüllung

Für die Mischung von Komponenten zur Herstellung von Arzneimitteln in einem Containmentsystem sollen beispielsweise in einem 60I fassenden Prozessbehälter 1, in dem sich ca. 20kg an pulverförmigen Stoffen einer oder mehrerer Hauptkomponenten befinden, beispielsweise Lactose oder Maisstärke, etwa 20g an pulverförmigen Aktivstoffen zugemischt werden.

Ein erstes Ausführungsbeispiel ist in Fig. 1 dargestellt. Die Vorrichtung nach Fig. 1 zeigt links einen Kupplungsflansch 2 und rechts eine Transporteinheit mit einer eingesteckten Kartusche 4. Die Transporteinheit dient gleichzeitig als Transportmittel vom Befüllkomplex zur Dosierstation für den Prozessbehälter 1.

Für kleinste zu dosierende Mengen werden dabei Kartuschen mit sehr kleinem Durchmesser verwendet. Dabei bestimmen der Hub und der Durchmesser des Förderkolbens 8 und der Innerdurchmesser der Pulverkammer 11 die Produktmenge, die aus der Kartusche dosiert oder entleert werden kann. Im hier beschriebenen Beispiel wird eine ca. 190mm lange Kartusche mit einem Innendurchmesser der Kartuschenhülse 12 von 34mm verwendet.

Die Transporteinheit weist einen Verbindungsflansch 6 auf, der aus Fertigungs- und Reinigungsgründen zweiteilig ausgebildet ist. Er besteht aus einer Kartuschenaufnahme 10 und einem Klappenventil 25 nach dem Prinzip gemäß EP 0 447 023 B1. Beide Teile sind mittels einer Klemme 20 lösbar miteinander verbunden. Die Kartuschenaufnahme 10 besitzt eine Bohrung zur Aufnahme der Kartusche 4. Durch diese Bohrung kann die genau gefüllte Kartusche 4 durch die geöffnete Verschlussklappe 7 des Klappenventiles 25 hindurch geschoben werden kann. In Fig. 1 ist die Kartusche 4 lediglich bis zur Verschlussklappe 7 geschoben, wobei die Verschlussklappe 7 und die Schutzhülle 19 den Verbindungsflansch 6 für den sicheren Transport dicht abschließt. Die Verschlussklappe 7 des Klappenventiles 25 ist mittels eines Hebelmechanismus 23 bedienbar.

Der Kupplungsflansch 2 bleibt als ständige Anschlusseinheit am Prozessbehälter 1. Der Kupplungsflansch 2 ist vorzugsweise zweiteilig ausgebildet, um einfache Fertigung und um eine bessere Reinigung beider Teile zu ermöglichen. Er besteht in diesem Ausführungsbeispiel aus einem Anschlussstück 17 und einem Klappenventil 24 nach dem Prinzip gemäß EP 0 447 023 B1. Beide Teile sind mittels einer Klemme 20 miteinander lösbar verbunden. Das Anschlussstück 17 ist so am Prozessbehälter 1 befestigt, dass die Innenseite Teil des Prozessbehälters ist. Es kann beispielsweise angeschweißt oder angeschraubt werden. Das Anschlussstück 17 besitzt eine Bohrung 22, bis zu dieser die Kartusche 4 durch die geöffnete Verschlussklappe 3 des Klappenventiles 24 hindurch bis an die innere Wandung des Prozessbehälters 1 geschoben werden kann. In Fig. 1 ist die Verschlussklappe 3 noch geschlossen dargestellt. Im vorliegenden Beispiel besitzt das Anschlussstück 17 einen Absatz 26, der als Anschlag für die Kartuschenhülse 12 dient. Der Durchmesser der Entleerungsöffnung 18 entspricht dem Außendurchmesser der Kartusche 4.

Die Verschlussklappen 3,7 der beiden Klappenventile 24,25 sind mittels des Hebelmechanismus 23 im zusammengebauten Zustand gemeinsam bedienbar. Nach dem Öffnen der Verschlussklappen 3,7 wird ober- und unterhalb der Verschlussklappen 3,7 ein Durchgang für die Kartusche 4 ermöglicht. Die beiden Bohrungen im Anschlussstück 17 sowie in der Kartuschenaufnahme 10 zur Aufnahme der Kartusche 4 müssen entsprechend korrespondieren. Dafür sorgen übliche Fixierungen zur Positionierung, beispielsweise Zentrierstifte.

Bei der Vorrichtung nach Fig. 1 erfolgt eine axiale Kupplung von zwei parallelen Flanschen nach dem in DE 20 2009 018 738 U1 beschriebenem Prinzip, wobei die Stirnflächen der beiden Flansche zum Kuppeln axial aneinander gepresst und mittels einer durch einen Betätigungshebel 27 bedienbaren Kupplungshülse 21 fixiert werden.

In Fig. 1 besteht die Kartusche 4 aus einer Kartuschenhülse 12, in deren inneren Pulverkammer 11 der Förderkolben 8 förderseitig mittels einer betätigbaren Kolbenstange 5 verschiebbar angeordnet ist. Die Kartusche 4 ist behälterseitig durch einen auf der gleichen Kolbenstange 5 angeordneten Verschlusskolben 15 gemeinsam verschließbar. Zum Schutz der Transporteinheit sowohl beim Transport als auch beim eigentlichen Dosiervorgang ist in die Kartuschenaufnahme 10 eine Nut eingearbeitet, in die mittels eines Halteringes 28 eine so große flexible Schutzhülle 19 befestigt werden kann, dass diese bis um den Griff 9 der ausgezogenen Kolbenstange 5 der Kartusche 4 reicht. Die Schutzhülle 19 besteht aus einer durchsichtigen Kunststofffolie.

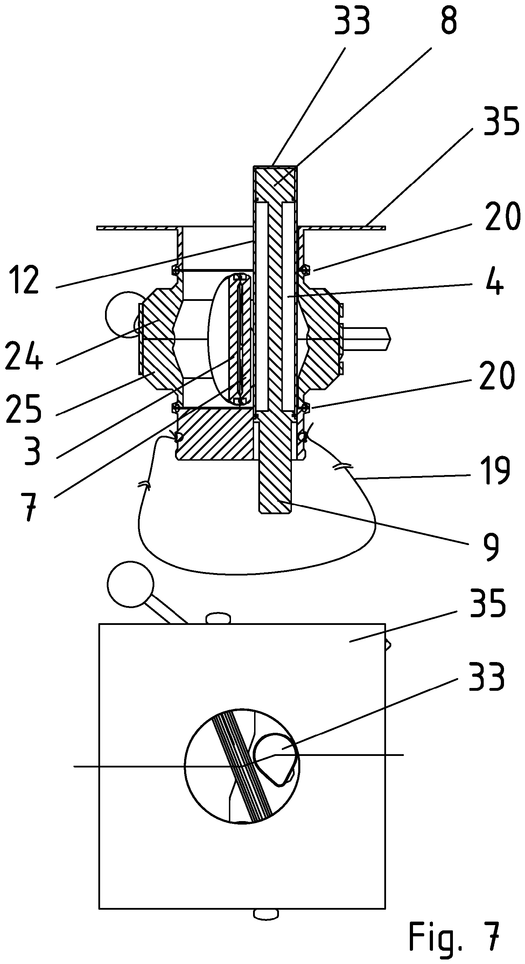

Die Funktionsweise ist wie folgt:
Nach dem in Fig. 7 beschriebenen separaten Befüllen der Kartusche 4 mit der benötigten Produktmenge wird die gesamte Transporteinheit in vorbeschriebener Weise an das korrespondierende Klappenventil 24 des Kupplungsflansches 2 angeschlossen und mit der Kupplungshülse 21 gesichert. Anschließend werden die beiden Verschlussklappen 3 und 7 geöffnet und die Kartusche 4 so weit bis in den Kupplungsflansch 2 hineingeschoben, dass die Kartuschenhülse 12 mit ihrem Anschlag 1 30 an dem Absatz 26 anschlägt. Die Kartuschenhülse 12 und die Vorderseite des Verschlusskolbens 15 verschließen die Bohrung 18 im Prozessbehälter 1. Anschließend können der Prozessstart und die Dosierung der Pulverhauptkomponente erfolgen.

Zur Dosierung des in der Kartusche 4 befindlichen Pulvers wird die Kolbenstange mit dem Verschlusskolben 15 und dem Förderkolben 8 mittels des Griffes 9 in Richtung Prozessbehälter gedrückt, bis der Griffanschlag 29 an der Kartuschenhülse 12 anschlägt und das vordere Ende des Förderkolbens 8 in der Ebene der inneren Wandung des Prozessbehälters 1 abschließt.

Die Kolbenstange 5 und der daran befindliche Verschlusskolben 15 ragen in den Prozessbehälter hinein. Da die Eintragungsstelle unterhalb des Füllstandes im Prozessbehälter 1 liegt, wird das zu dosierende Pulver vom bereits im Prozessbehälter 1 befindlichem Pulvergemisch der Hauptkomponenten im Mischprozess, der im Prozessbehälter stattfindet, mitgerissen und homogen verteilt.

Nach der Materialzugabe und dem beendeten Mischprozess sowie der vollständigen Entleerung des Prozessbehälters 1 wird die Kolbenstange 5 wieder in die Kartusche 4 zurückgezogen. Danach wird die Kartusche 4 zurückgezogen, bis der Anschlag 2 31 an der Kartuschenaufnahme 10 anschlägt und die beiden Verschlussklappen 3 und 7 verschlossen werden können.

Nach dem Entkoppeln der beiden Flansche wird die Transporteinheit zur Wiederbefüllung der Kartusche 4 abtransportiert.

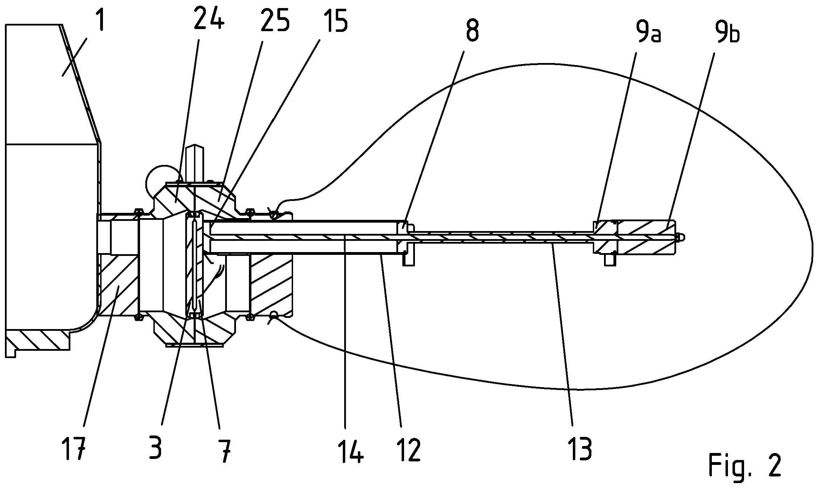

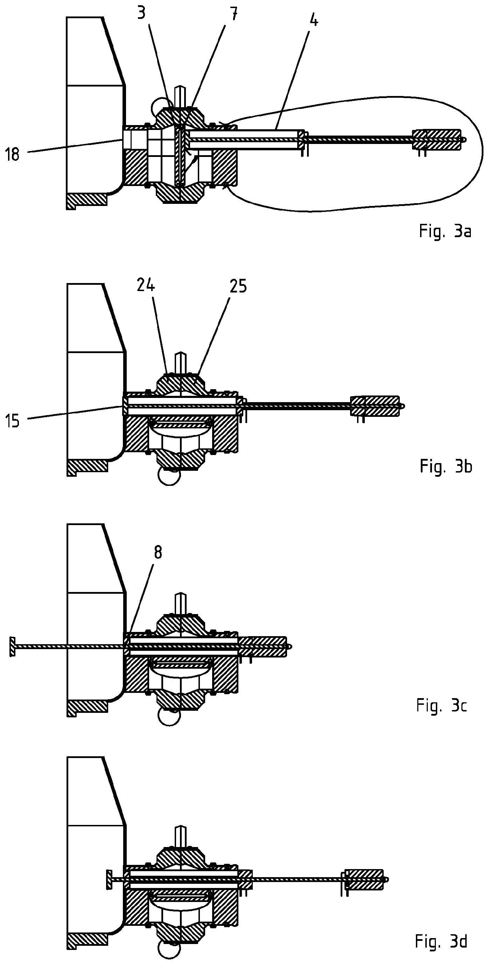

Ein weiteres Ausführungsbeispiel ist in Fig. 2 und den Fig. 3a bis 3d dargestellt. Die Fig. 2 zeigt eine Kartusche 4 mit einer Doppelkolbenlösung.

Die Befüllung der Kartusche 4 und die Einbringung in die Transporteinheit durch die beiden Klappenventile 25 und 24 und das Anschlussstück 17 bis zur inneren Wandung des Prozessbehälters erfolg analog dem Ausführungsbeispiel nach Fig. 1. Die verwendete Kartusche 4 besitzt innerhalb ihrer Kartuschenhülse 12 wieder einen Verschlusskolben 15 sowie einen Förderkolben 8. Der Unterschied besteht jedoch nun darin, dass beide Kolben mit jeweils einer eigenen Kolbenstange betätigt werden können. Im dargestellten Beispiel ist der innere Verschlusskolben 15 an der inneren Kolbenstange 2 14 befestigt und mittels des rechten Griffteiles 9b bedienbar. Der Förderkolben 8 ist an der äußeren Kolbenstange 1 befestigt und mittels des linken Griffteiles 9a bedienbar.

Die Figuren 3a bis 3d zeigen die Funktionsweise einer in Fig. 2 beschriebenen Doppelkolbenlösung.

In Fig. 3a ist die befüllte und mit dem Verschlusskolben 15 verschlossene Kartusche 4 bis an die Verschlussklappe 7 eingeführt.

In Fig. 3b durchstößt die Kartusche 4 bei geöffneten Verschlussklappen 3 und 7 die Klappenventile 25 und 24. Der Verschlusskolben 15 verschließt dabei noch die Entleerungsöffnung 18. Die beiden Kolben befinden sich noch in der gleichen Stellung wie in Fig. 3a.

Anschließend kann die Entleerung der Kartusche 4 durch gemeinsames Einschieben der beiden Kolben erfolgen, bis das vordere Ende des Förderkolbens 8 in der Ebene der inneren Wandung des Prozessbehälters 1 abschließt. In Fig. 3c ist die Kartusche 4 in dieser Stellung bereits geleert. Es ist auch möglich, den Verschlusskolben 15 nur ein definiertes Stück einzuschieben und dann mit dem Förderkolben 8 die Kartusche 4 zu entleeren. Ein Zurückziehen des Verschlusskolbens 15 ist dann, wie in Fig. 3d gezeigt, nicht mehr nötig.

In Fig. 3d ist zu sehen, dass der Verschlusskolben 15 nach der Entleerung getrennt vom Förderkolben 8 zurückgezogen werden kann. Der Verschlusskolben 15 darf bis kurz vor den Förderkolben 8 nur so weit zurückgezogen werden, damit keine Produktmengen zwischen den Kolben verbleiben. In dieser Stellung wird der Prozess beendet, der Prozessbehälter 1 entleert und die Kartusche 4 wie in Fig. 1 beschrieben zurückgezogen.

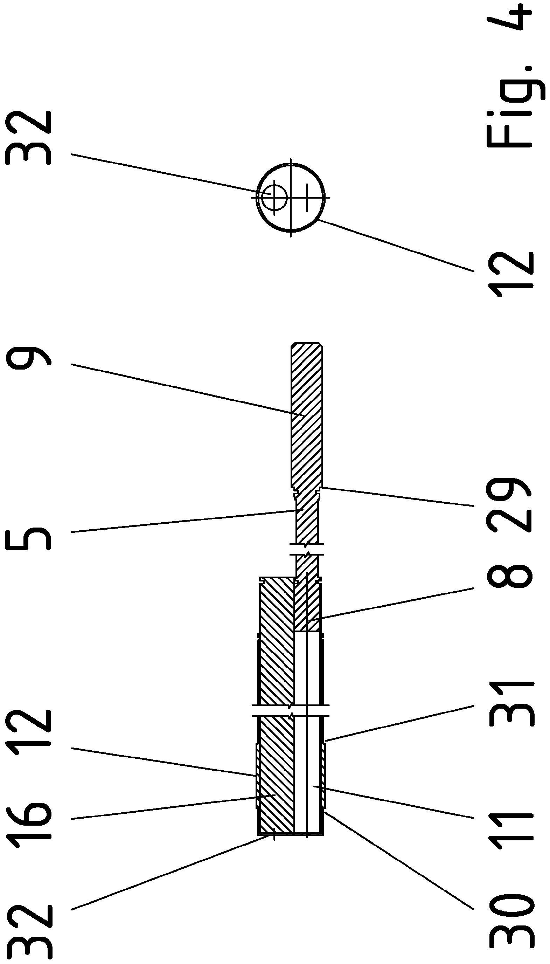

In Fig. 4 ist eine besondere Ausführung für eine Kartusche 4 dargestellt. Innerhalb einer Kartuschenhülse 12 befindet sich ein um mindestens 100° axial verdrehbarer Kartuschenkern 16. Die Pulverkammer 11 erstreckt sich über die gesamte Länge des Kartuschenkernes 16. Der Förderkolben 8 ist mittels der Kolbenstange 5 verschiebbar in der Pulverkammer 11 angeordnet. Die Fig. 4 zeigt die Kartusche 4 im geschlossenen Zustand. Zur Entleerung der Kartusche 4 wird der Kartuschenkern 16 so weit verdreht, bis die Pulverkammer 11 mit der in der Stirnseite der Kartuschenhülse 12 befindlichen Öffnung 32 übereinstimmt und das enthaltene Pulvermaterial in den Prozessbehälter freigibt. Der Förderkolben 8 wird bis zum Griffanschlag 29 an den Kartuschenkern 16 geschoben, dass das vordere Ende des Förderkolbens 8 in der Ebene der inneren Wandung des Prozessbehälters 1 abschließt.

Die hier beschriebene Kartusche 4 besitzt ebenfalls eine Länge von 190mm und einen Innendurchmesser der Kartuschenhülse 12 von 34mm. Da die Pulverkammer 11 jedoch nur einen Durchmesser von 12mm besitzt, ist eine exaktere Dosierung von wesentlich kleineren Dosiermengen erreichbar.

Die Kartusche 4 ist wie in den vorgenannten Beispielen in die Transporteinheit und den Kupplungsflansch einsetzbar und nutzbar.

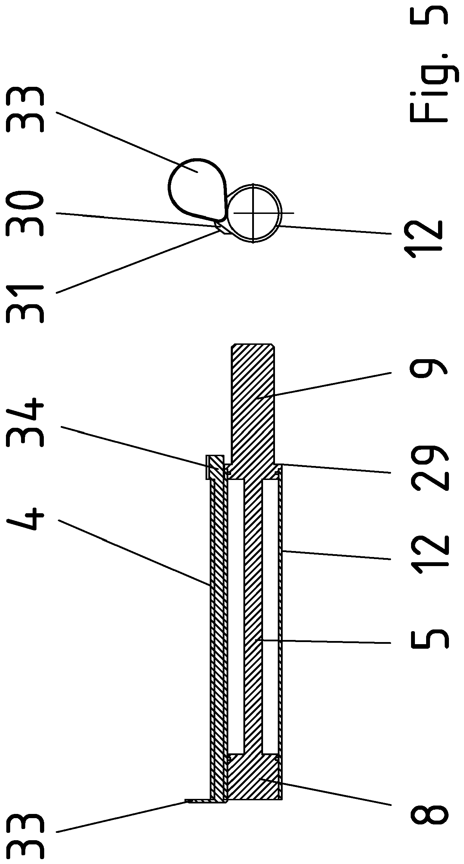

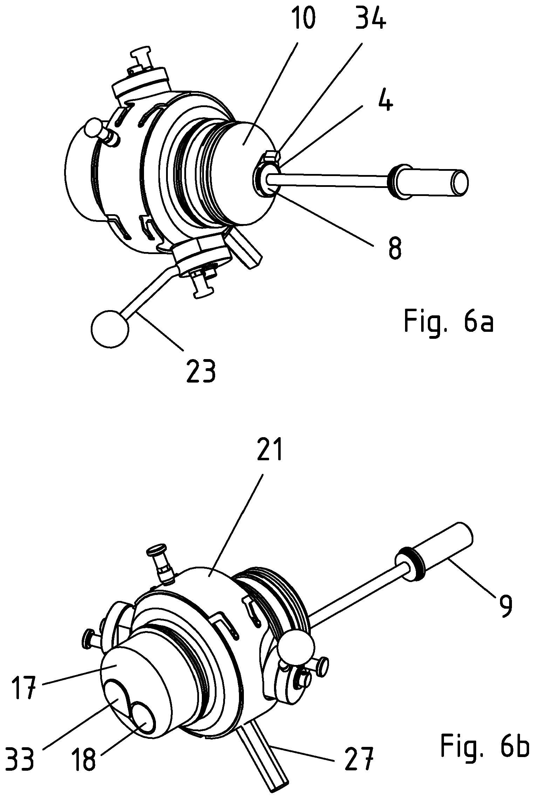

Eine weitere Ausführung der erfindungsgemäßen Vorrichtung ist aus den Fig. 5 sowie 6a und 6b ersichtlich. Hier ist eine Variante dargestellt, bei der im Inneren der Kartuschenhülse 12 in vorbeschriebener Weise ein runder Förderkolben 8 mittels einer Kolbenstange 5 und einem daran befestigten Griff 9 verschiebbar angeordnet ist. Am Griff 9 befindet sich ein Griffanschlag 29, der so positioniert ist, dass der eingeschobene Förderkolben 8, wie in Fig. 5 zu sehen, im entleerten Zustand mit seinem vorderen Ende in der Ebene der inneren Wandung des Prozessbehälters 1 abschließt.

Die hier dargestellte rohrförmige Kartusche 4 besitzt jedoch eine ovale Kartuschenhülse 12, in deren seitlichen Ausformung ein Drehverschluss 33 integriert wird. Der Drehverschluss besteht aus einer in die seitliche Ausformung integrierte Welle, an deren vorderen Ende eine Verschlussklappe vorgesehen ist. Am hinteren Ende der Welle befindet sich ein Betätigungshebel 34, mittels dem die Verschlussklappe über die Entleerungsöffnung 18 geschwenkt werden und diese vollständig verschließen kann.

Zur Realisierung dieser Ausführungsform ist es erforderlich, dass sowohl das Anschlussstück 17 als auch die Kartuschenaufnahme 10 eine innere ovale Form besitzen müssen, die der Kontur der Kartuschenhülse 12 entspricht und das Einschieben der Kartusche 4 ermöglicht.

Diese Ausführung hat den Vorteil, dass ein gesonderter Verschlusskolben nicht mehr erforderlich ist. Nach der Dosierung verbleibt damit kein Verschlusskolben im Prozessbehälter und die Entleerungsöffnung 18 kann verschlossen werden.
Die Kartusche 4 kann auch bei dieser Ausführung erst nach Entleerung des Prozessbehälters 1 entnommen werden.

In Fig. 7 wird eine an einen Befüllkomplex angeschlossene Transporteinheit dargestellt, bei der eine Kartusche mit einem Drehverschluss 33 nach Fig. 5 verwendet wird. In Fig. 7 ist nicht dargestellt, dass der Befüllkomplex aus einer Glovebox, einem Wägesystem mit einer Kartuschenaufnahme und einem Mikrodosiersystem zur Befüllung besteht.
Am Befüllkomplex ist ein fester Anschlussflansch 35 vorhanden, an dem ein in vorbenannter Weise ausgebildetes Klappenventil 24 mittels einer Klemme 20 befestigt wurde. Nach dem Ankoppeln der Transporteinheit werden die beiden Verschlussklappen 3 und 7 über ihren Hebelmechanismus geöffnet. Da der Anschlussflansch 35 am Befüllsystem keinen Absatz 26 aufweist, kann die entleerte Kartusche 4 anschließend durch die beiden Verschlussklappen 3 und 7 der Klappenventile 25 und 24 in den Befüllkomplex hinein geschoben und entnommen werden. In Fig. 7 wird die entleerte Kartusche 4 bei geschlossenem Drehverschluss 33 gerade aus der Transporteinheit in den Befüllkomplex geschoben. Dort kann sie unter Containmentbedingungen gereinigt und wieder befüllt werden. Die Rückführung der gefüllten Kartusche 4 in die Transporteinheit erfolgt in umgekehrter Reihenfolge.

### Bezugszeichenliste

- 1: Prozessbehälter
- 2: Kupplungsflansch
- 3: Verschlussklappe
- 4: Kartusche
- 5: Kolbenstange
- 6: Verbindungsflansch
- 7: Verschlussklappe
- 8: Förderkolben
- 9: Griff
- 9a: Griffteil
- 9b: Griffteil
- 10: Kartuschenaufnahme
- 11: Pulverkammer
- 12: Kartuschenhülse
- 13: Kolbenstange 1
- 14: Kolbenstange 2
- 15: Verschlusskolben
- 16: Kartuschenkern
- 17: Anschlussstück
- 18: Entleerungsöffnung
- 19: Schutzhülle
- 20: Klemme
- 21: Kupplungshülse
- 22: Bohrung
- 23: Hebelmechanismus
- 24: Klappenventil
- 25: Klappenventil
- 26: Absatz
- 27: Betätigungshebel
- 28: Haltering
- 29: Griffanschlag
- 30: Anschlag 1
- 31: Anschlag 2
- 32: Öffnung
- 33: Drehverschluss
- 34: Betätigungshebel
- 35: Anschlussflansch

## Patentansprüche

1. Vorrichtung zum Einbringen einer definierten Menge eines zweiten Pulvers in einen Prozessbehälter (1), in dem sich ein erstes Pulver oder Pulvergemisch befindet, wobei die Vorrichtung den Prozessbehälter (1), eine rohrförmige Kartusche (4) und eine Transporteinheit umfasst, wobei das zweite Pulver in die rohrförmige Kartusche (4) einbringbar ist, und wobei die Kartusche (4) verschiebbar in der Transporteinheit lagerbar ist, **dadurch gekennzeichnet,**
- **dass** am Prozessbehälter (1) ein Kupplungsflansch (2) mit einer Verschlussklappe (3) vorhanden ist,
- **dass** die Transporteinheit einen Verbindungsflansch (6) mit einer Verschlussklappe (7) aufweist,
- **dass** der Verbindungsflansch (6) mit dem Kupplungsflansch (2) verbindbar ist, derart, dass die Verschlussklappe (3) des Prozessbehälters und die Verschlussklappe (7) der Transporteinheit geöffnet werden können,
- **dass** die Kartusche (4) innerhalb der Transporteinheit durch die geöffneten Verschlussklappen (3 und 7) hindurch bis an die Ebene der inneren Wandung des Prozessbehälters (1) verschiebbar ist und
- **dass** das zweite Pulver mittels eines Förderkolbens (8) der Kartusche (4) aus der Kartusche (4) in den Prozessbehälter (1) entleerbar ist.

2. Vorrichtung nach Anspruch 1, **dadurch gekennzeichnet, dass** der Kupplungsflansch (2) aus einem Anschlussstück (17) und einem Klappenventil (24) besteht.

3. Vorrichtung nach Anspruch 1 oder 2, **dadurch gekennzeichnet, dass** der Verbindungsflansch (6) aus einer Kartuschenaufnahme (10) und einem Klappenventil (25) besteht.

4. Vorrichtung nach einem der Ansprüche 1 bis 3, **dadurch gekennzeichnet, dass** die Kartusche (4) aus einer Kartuschenhülse (12) besteht, in deren innerer Pulverkammer (11) der Förderkolben (8) mittels einer Kolbenstange (5) verschiebbar angeordnet ist und die Kartusche (4) behälterseitig durch einen mittels der betätigbaren Kolbenstange (5) verschiebbar angeordneten Verschlusskolben (15) verschließbar ist.

5. Vorrichtung nach einem der Ansprüche 1 bis 3, **dadurch gekennzeichnet, dass** die Kartusche (4) aus einer Kartuschenhülse (12) besteht, in deren innerer Pulverkammer (11) der Förderkolben (8) mittels einer Kolbenstange 1 (13) verschiebbar angeordnet ist und die Kartusche (4) behälterseitig durch einen mittels einer zweiten Kolbenstange 2 (14) verschiebbar angeordneten Verschlusskolben (15) verschließbar ist.

6. Vorrichtung nach einem der Ansprüche 1 bis 3, **dadurch gekennzeichnet, dass** die Kartusche (4) aus einer Kartuschenhülse (12) besteht, in der ein um mindestens 100° axial verdrehbarer Kartuschenkern (16) angeordnet ist,
- sich über die Länge des Kartuschenkernes (16) eine Pulverkammer (11) erstreckt, in die der Förderkolben (8) mittels einer Kolbenstange (5) verschiebbar angeordnet ist,
- die Kartuschenhülse (12) behälterseitig eine Entleerungsöffnung (32) aufweist und zum Entleeren der Kartusche (4) der Kartuschenkern (16) so weit gedreht wird, bis die Pulverkammer (11) mit der Entleerungsöffnung (32) korreliert.

7. Vorrichtung nach einem der Ansprüche 1 bis 3, **dadurch gekennzeichnet, dass** die Kartusche (4) aus einer Kartuschenhülse (12) besteht, in die seitlich ein Drehverschluss (33) integrierbar ist, mit dem die Entleerungsöffnung der Kartuschenhülse (12) mittels eines Betätigungshebels (34) verschließbar ist.

8. Vorrichtung nach einem der Ansprüche 1 bis 7, **dadurch gekennzeichnet, dass** am Verbindungsflansch (6) eine flexible Schutzhülle (19) so befestigt ist, dass die Schutzhülle (19) die eingeschobene Kartusche (4) vollständig umschließt.

9. Vorrichtung nach einem der Ansprüche 1 bis 8, **dadurch gekennzeichnet, dass** bei verbundenem Verbindungsflansch (6) und Kupplungsflansch (2) die Verschlussklappen (3,7) zueinander dicht abschließen und gemeinsam schwenkbar sind.

10. Vorrichtung nach einem der Ansprüche 1 bis 3, **dadurch gekennzeichnet, dass** die Kartusche (4) an ihrer Entleerungsöffnung mit einer durchstoßbaren Membran verschlossen ist.

11. Vorrichtung nach Anspruch 10, **dadurch gekennzeichnet, dass** die Membran aus einem Material besteht, welches eine Komponente der im Prozessbehälter (1) zu mischenden Pulver ist.

12. Vorrichtung nach einem der Ansprüche 1 bis 11, **dadurch gekennzeichnet, dass** der Förderkolben (8) über eine lösbare Verbindung mit einem Antrieb gekoppelt ist, der eine automatische Dosierung oder Entleerung bewirkt.

## Claims

1. Apparatus for introducing a defined quantity of a second powder into a process vessel (1) in which a first powder or powder mixture is located, wherein the apparatus comprises the process vessel (1), a tubular cartridge (4) and a transport unit, wherein the second powder can be introduced into the tubular cartridge (4), and wherein the cartridge (4) can be mounted displaceably in the transport unit, **characterized in that**
- a coupling flange (2) comprising a closure flap (3) is present on the process vessel (1),
- the transport unit has a connecting flange (6) comprising a closure flap (7),
- the connecting flange (6) is connectable to the coupling flange (2) in such a way that the closure flap (3) of the process vessel and the closure flap (7) of the transport unit can be opened,
- the cartridge (4) inside the transport unit is displaceable through the opened closure flaps (3 and 7) as far as the plane of the inner wall of the process vessel (1), and
- the second powder can be emptied out of the cartridge (4) into the process vessel (1) by means of a delivery piston (8) of the cartridge (4) .

2. Apparatus according to Claim 1, **characterized in that** the coupling flange (2) is composed of an attachment piece (17) and a flap valve (24).

3. Apparatus according to Claim 1 or 2, **characterized in that** the connecting flange (6) is composed of a cartridge holder (10) and a flap valve (25).

4. Apparatus according to one of Claims 1 to 3, **characterized in that** the cartridge (4) is composed of a cartridge sleeve (12) having an inner powder chamber (11) in which the delivery piston (8) is arranged to be displaceable by means of a piston rod (5), and the cartridge (4) is closable, at the vessel side, by a closure piston (15) which is arranged to be displaceable by means of the actuatable piston rod (5).

5. Apparatus according to one of Claims 1 to 3, **characterized in that** the cartridge (4) is composed of a cartridge sleeve (12) having an inner powder chamber (11) in which the delivery piston (8) is arranged to be displaceable by means of a piston rod 1 (13), and the cartridge (4) is closable, at the vessel side, by a closure piston (15) which is arranged to be displaceable by means of a second piston rod 2 (14).

6. Apparatus according to one of Claims 1 to 3, **characterized in that** the cartridge (4) is composed of a cartridge sleeve (12) in which a cartridge core (16) is arranged rotatably about an axis by at least 100°,
- a powder chamber (11) extends over the length of the cartridge core (16), in which powder chamber (11) the delivery piston (8) is arranged to be displaceable by means of a piston rod (5),
- the cartridge sleeve (12) has, at the vessel side, a discharge opening (32), and, in order to empty the cartridge (4), the cartridge core (16) is rotated until the powder chamber (11) aligns with the discharge opening (32).

7. Apparatus according to one of Claims 1 to 3, **characterized in that** the cartridge (4) is composed of a cartridge sleeve (12) in which a rotary closure piece (33) can be integrated laterally, and the discharge opening of the cartridge sleeve (12) can be closed with the rotary closure piece (33) by means of an actuating lever (34).

8. Apparatus according to one of Claims 1 to 7, **characterized in that** a flexible protective sheath (19) is fastened to the connecting flange (6) such that the protective sheath (19) completely encloses the inserted cartridge (4).

9. Apparatus according to one of Claims 1 to 8, **characterized in that**, with connecting flange (6) and coupling flange (2) connected, the closure flaps (3, 7) seal tightly with each other and are pivotable together.

10. Apparatus according to one of Claims 1 to 3, **characterized in that** the cartridge (4) is closed, at its discharge opening, with a pierceable membrane.

11. Apparatus according to Claim 10, **characterized in that** the membrane is made of a material that is a component of the powders that are to be mixed in the process vessel (1).

12. Apparatus according to one of Claims 1 to 11, **characterized in that** the delivery piston (8) is coupled, via a releasable connection, to a drive which effects automatic metering or emptying.

## Revendications

1. Dispositif d'introduction d'une quantité précise d'une deuxième poudre dans un conteneur (1) dans lequel se trouve une première poudre ou un premier mélange de poudres, le dispositif comprenant le conteneur (1), une cartouche tubulaire (4) et une unité de transport, la deuxième poudre pouvant être introduite dans la cartouche tubulaire (4), et la cartouche (4) pouvant être logée de manière coulissante dans l'unité de transport, **caractérisé en ce que**
- une bride d'accouplement (2) pourvue d'un volet de fermeture (3) est présente au niveau du conteneur (1),
- l'unité de transport comporte une bride de liaison (6) pourvue d'un volet de fermeture (7),
- la bride de liaison (6) peut être reliée à la bride d'accouplement (2) de telle sorte que le volet de fermeture (3) du conteneur et le volet de fermeture (7) de l'unité de transport puissent être ouverts,
- la cartouche (4) peut coulisser à l'intérieur de l'unité de transport à travers les volets de fermeture ouverts (3 et 7) jusqu'au plan de la paroi intérieure du conteneur (1) et
- la deuxième poudre peut être vidée de la cartouche (4) dans le conteneur (1) au moyen d'un piston de refoulement (8) de la cartouche (4).

2. Dispositif selon la revendication 1, **caractérisé en ce que** la bride d'accouplement (2) comprend une pièce de raccordement (17) et une soupape de volet (24) .

3. Dispositif selon la revendication 1 ou 2, **caractérisé en ce que** la bride de liaison (6) comprend un logement de cartouche (10) et une soupape de volet (25) .

4. Dispositif selon l'une des revendications 1 à 3, **caractérisé en ce que** la cartouche (4) comprend un manchon de cartouche (12) dans la chambre à poudre intérieure (11) duquel le piston de refoulement (8) est disposé de manière coulissante au moyen d'une tige de piston (5) et la cartouche (4) peut être fermée côté récipient par un piston de fermeture (15) qui est disposé de manière coulissante au moyen de la tige de piston actionnable (5).

5. Dispositif selon l'une des revendications 1 à 3, **caractérisé en ce que** la cartouche (4) comprend un manchon de cartouche (12) dans la chambre à poudre intérieure (11) duquel le piston de refoulement (8) est disposé de manière coulissante au moyen d'une tige de piston 1 (13) et la cartouche (4) peut être fermée côté récipient par un piston de fermeture (15) disposé de manière coulissante au moyen d'une deuxième tige de piston 2 (14).

6. Dispositif selon l'une des revendications 1 à 3, **caractérisé en ce que** la cartouche (4) comprend un manchon de cartouche (12) dans lequel est disposé un noyau de cartouche (16) pouvant être tourné axialement d'au moins 100°,
- une chambre à poudre (11), dans laquelle le piston de refoulement (8) est disposé de manière coulissante au moyen d'une tige de piston (5), s'étend sur la longueur du noyau de cartouche (16),
- le manchon de cartouche (12) comporte côté récipient une ouverture de vidage (32) et, pour vider la cartouche (4), le noyau de cartouche (16) est mis en rotation jusqu'à ce que la chambre à poudre (11) soit en liaison avec l'ouverture de vidage (32).

7. Dispositif selon l'une des revendications 1 à 3, **caractérisé en ce que** la cartouche (4) comprend un manchon de cartouche (12) dans lequel un verrou rotatif (33) peut être intégré latéralement, lequel permet de fermer l'ouverture de vidage du manchon de cartouche (12) à l'aide d'un levier d'actionnement (34).

8. Dispositif selon l'une des revendications 1 à 7, **caractérisé en ce qu'**une gaine de protection souple (19) est fixée sur la bride de liaison (6) de sorte que la gaine de protection (19) enferme complètement la cartouche insérée (4).

9. Dispositif selon l'une des revendications 1 à 8, **caractérisé en ce que**, lorsque la bride de liaison (6) et la bride d'accouplement (2) sont reliées, les volets de fermeture (3, 7) sont verrouillés de manière étanche l'un à l'autre et peuvent pivoter conjointement.

10. Dispositif selon l'une des revendications 1 à 3, **caractérisé en ce que** la cartouche (4) est fermée au niveau de son ouverture de vidage par une membrane perforable.

11. Dispositif selon la revendication 10, **caractérisé en ce que** la membrane est en un matériau qui est un composant de la poudre à mélanger dans le conteneur (1) .

12. Dispositif selon l'une des revendications 1 à 11, **caractérisé en ce que** le piston de refoulement (8) est accouplé par une liaison amovible à un entraînement qui effectue un dosage ou un vidage automatique.
